(19) Europäisches Patentamt

European Patent Office

Office européen des brevets

(11) Veröffentlichungsnummer : **0 350 659 B1**

# (12) EUROPÄISCHE PATENTSCHRIFT

(45) Veröffentlichungstag der Patentschrift :
23.09.92 Patentblatt 92/39

(51) Int. Cl.⁵ : **C07C 7/08, B01D 3/40**

(21) Anmeldenummer : **89111093.4**

(22) Anmeldetag : **19.06.89**

(54) Verfahren zur Trennung von paraffinischen und olefinischen C4-Kohlenwasserstoffen.

(30) Priorität : **14.07.88 DE 3823772**

(43) Veröffentlichungstag der Anmeldung :
**17.01.90 Patentblatt 90/03**

(45) Bekanntmachung des Hinweises auf die
Patenterteilung :
**23.09.92 Patentblatt 92/39**

(84) Benannte Vertragsstaaten :
**DE FR GB**

(56) Entgegenhaltungen :
**DE-A- 3 532 289**
**US-A- 2 588 056**

(73) Patentinhaber : **Krupp Koppers GmbH**
**Altendorfer Strasse 120**
**W-4300 Essen 1 (DE)**

(72) Erfinder : **Emmrich, Gerd**
**Kamperfield 21**
**W-4300 Essen 1 (DE)**
Erfinder : **Schneider, Hans-Christoph**
**Rosental 8**
**W-4320 Hattingen (DE)**

EP 0 350 659 B1

**Beschreibung**

Die Erfindung betrifft ein Verfahren zur Trennung von paraffinischen und olefinischen $C_4$-Kohlenwasserstoffen durch Extraktivdestillation unter Verwendung von wasserfreiem Morpholin als selektives Lösungsmittel, wobei die Olefine zusammen mit der Hauptmenge des Lösungsmittels aus dem Sumpf der Extraktivdestillationskolonne abgezogen und in einer nachgeschalteten Abtriebskolonne vom Lösungsmittel abgetrennt werden, während die Paraffine als Kopfprodukt aus der Extraktivdestillationskolonne entfernt werden.

Aus den auf der Basis von Naphtha als Einsatzprodukt arbeitenden Äthylen-Steamcrackern sowie in katalytischen Crackanlagen fallen in der chemischen Industrie große Mengen an $C_4$-Fraktionen an, die neben stärker ungesättigten $C_4$-Kohlenwasserstoffen insbesondere $C_4$-Paraffine und $C_4$-Monoolefine enthalten. Auf Grund ihrer hohen Reaktionsfähigkeit werden dabei die $C_4$-Monoolefine im zunehmenden Maße als Ausgangsstoffe für weiterführende Reaktionen eingesetzt. Aus diesem Grunde ist das Interesse der Fachwelt an einer wirtschaftlichen Auftrennung der anfallenden $C_4$-Fraktionen ständig gewachsen. Es sind deshalb bereits Verfahrensgänge entwickelt worden, bei denen zunächst aus der eingesetzten $C_4$-Fraktion das Butadien durch Gaswäsche oder Extraktivdestillation mit speziellen Lösungsmitteln abgetrennt wird, während das Isobuten durch Selektivreaktionen wie Schwefelsäureesterbildung oder die Herstellung von Methyl-tert.-Butylether (MTB) aus dem Einsatzprodukt entfernt wird. Die danach im Einsatzprodukt verbleibenden Komponenten Isobutan, n-Butan, Buten-1 und cis/trans-Buten 2 sind auf Grund ihrer Siedelage nur noch schwer, zum Teil aber auch gar nicht auf rein destillativem Wege zu trennen. Es besteht deshalb derzeit bei der Fachwelt ein ausgesprochenes Bedürfnis nach Verfahren, die die Trennung der genannten Verbindungen auf einfache und besonders wirtschaftliche Weise ermöglichen.

In diesem Zusammenhang ist bereits vorgeschlagen worden, die Trennung von paraffinischen und olefinischen $C_4$-Kohlenwasserstoffen durch Extraktivdestillation unter Verwendung eines selektiven Lösungsmittels vorzunehmen. So ist beispielsweise aus der EP-B1-216 991 ein Verfahren der eingangs genannten Art bekannt, bei dem als selektives Lösungsmittel wasserfreies Morpholin zur Anwendung gelangt.

In der US-A-2 588 056 wird ferner ein Verfahren zur Trennung von Propen und Propan durch Extraktivdestillation beschrieben, bei dem Acetonitril oder Kohlenwasserstoffe aus der Gruppe der Acetonitrile oder Chloracetonitrile als selektives Lösungsmittel verwendet werden sollen. Dabei wird vorgeschlagen, das als Kopfprodukt der Extraktivdestillation anfallende Propan in einer am Kopf der Extraktivdestillationskolonne angeordneten und als Dephlegmator bezeichneten Kolonne von Lösungsmittelresten zu befreien.

Das heißt, bei der Durchführung derartiger Extraktivdestillationen war es bisher üblich, daß die Paraffine, die als Raffinat über Kopf aus der Extraktivdestillationskolonne abgezogen werden, in einer separaten Kolonne durch Destillation von den in ihnen noch enthaltenen Lösungsmittelresten abgetrennt werden. Es liegt auf der Hand, daß eine derartige Kolonne zur Raffinataufarbeitung zusätzliche Betriebs- und Anlagekosten verursacht.

Der Erfindung lag deshalb die Aufgabe zugrunde, das Verfahren der eingangs genannten Art weiter zu optimieren und insbesondere die Raffinataufarbeitung zu vereinfachen.

Das der Lösung dieser Aufgabe dienende Verfahren der eingangs genannten Art ist erfindungsgemäß dadurch gekennzeichnet, daß die in den Paraffinen enthaltenen Lösungsmittelreste in einem mit der Extraktivdestillationskolonne einebauliche Einheit bildenden und ebenfalls mit Böden oder sonstigen Einbauten versehenen Kolonnenaufsatz aus den Paraffinen entfernt werden, wobei der Kolonnenaufsatz mit einem Rückflußverhältnis von 1 bis 2 betrieben wird.

Das heißt, beim erfindungsgemäßen Verfahren wird auf eine Behandlung der Paraffine in einer separaten Kolonne verzichtet. Stattdessen wird die Extraktivdestillationskolonne oberhalb des Lösungsmittelaufgabebodens mit einem zusätzlichen Kolonnenaufsatz versehen, der nur wenige Böden, beispielsweise 10, aufzuweisen braucht. Dieser Kolonnenaufsatz reicht bei einem Rückflußverhältnis von 1 bis 2 aus, um die in den Paraffinen enthaltenen Lösungsmittelreste zurückzuhalten.

Dieses Ergebnis, das im Hinblick auf die bisher geübte Praxis als überraschend angesehen werden muß, läßt sich im wesentlichen mit der besonderen Zusammensetzung der in diesem Falle als Einsatzprodukt zur Anwendung gelangenden $C_4$-Fraktionen erklären. Diese $C_4$-Fraktionen enthalten nur paraffinische und olefinische $C_4$-Kohlenwasserstoffe, wobei das n-Butan die schwerste Komponente darstellt. Im Gegensatz zur Aufarbeitung anderer Kohlenwasserstoffgemische durch Extraktivdestillation, wie z.B. der Aromatengewinnung, fehlt hier im Einsatzprodukt eine kritische Schlüsselkomponente, wie z.B. Methylcyclohexan, die sich durch Rückfluß im Oberteil der Extraktivdestillationskolonne aufkonzentrieren und dann entweder in den Kolonnensumpf durchschlagen und dort die Reinheit des Extraktives verschlechtern oder sich in dem Maße unterhalb der Lösungsmittelaufgabe anreichern würde, daß dadurch eine Entmischung von Lösungsmittel- und Raffinaphase auftritt. Im Gegensatz dazu läßt sich das n-Butan jedoch zusammen mit dem iso-Butan problemlos über Kopf aus der Extraktivdestillationskolonne abdestillieren und zeigt deshalb die vorstehend beschriebenen Erscheinungen nicht.

EP 0 350 659 B1

Es liegt auf der Hand, daß durch die erfindungsgemäße Verwendung eines Kolonnenaufsatzes auf der Extraktivdestillationsklonne anstelle einer separaten Kolonne die Betriebs- und Anlagekosten für die Raffinatbehandlung nicht unbeträchtlich verringert werden und der Verfahrensablauf insgesamt vereinfacht wird.

Das bei der Durchführung des erfindungsgemäßen Verfahrens als Lösungsmittel verwendete wasserfreie Morpholin zeichnet sich einerseits für den genannten Zweck durch besonders gute Selektivität und gute Lösungsmitteleigenschaften aus, so daß auch bei niedrigen Olefinkonzentrationen im Einsatzprodukt hohe Olefinausbeuten in der Größenordnung von > 99 % erreicht werden können. Andererseits kann, da das Lösungsmittel ohne jeden Wasserzusatz angewandt wird, auf eine nachträgliche Entwässerung (Trocknung) sowohl des Raffinates als auch des Extraktes verzichtet werden, so daß beide Produkte ohne weiteres in wasserempfindlichen Reaktionen weiter umgesetzt werden können.

Als Einsatzprodukt für das erfindungsgemäße Verfahren kommen, wie bereits weiter oben gesagt wurde, Paraffine und Olefine enthaltende $C_4$-Fraktionen in Frage, wie sie bei verschiedenen technischen Prozessen anfallen. Typische Beispiele sind die bereits eingangs erwähnten Gemische, die nach der Entfernung des Butadiens sowie des Isobutens aus $C_4$-Fraktionen der Äthylen-Erzeugung oder der katalytischen bzw. thermischen Crackung von Kohlenwasserstoffen anfallen. Derartige Gemische enthalten, wie bereits erwähnt wurde, insbesondere Iso- und n-Butan, Buten-1 sowie cis/trans-Buten-2. Es gibt daneben aber auch technische Aufarbeitungswege, bei denen Gemische anfallen, in denen neben iso- und n-Butan nur cis/trans-Buten-2 oder nur n-Butan und cis/trans-Buten-2 enthalten sind. Die gemeinsame Gewinnung von Buten-1 und cis/trans-Buten-2 aus diesen Gemischen ist vor allem in den Fällen von Interesse, bei denen die drei Olefine als gleichrangige Reaktionspartner reagieren, was z.B. bei der weiteren Umsetzung zu Methyläthylketon der Fall ist. Auch die Isolierung von cis/trans-Buten-2 aus Gemischen mit n-Butan ist von besonderem Interesse, beispielsweise für die Oligomerisierung zu vorzugsweise Octen. Für alle diese Trennungen ist das erfindungsgemäße Verfahren besonders gut geeignet.

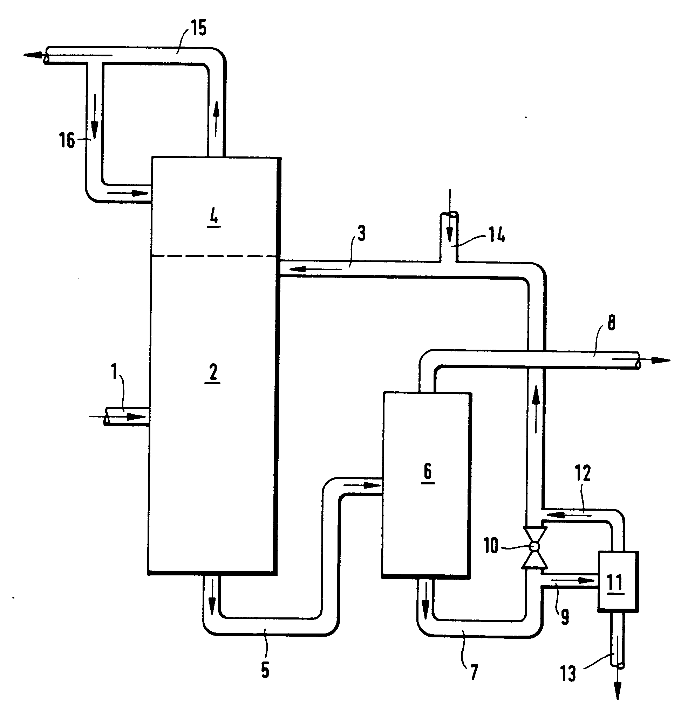

Für die Durchführung dieses Verfahrens können die für Extraktivdestillationen üblichen und allgemein gebräuchlichen Apparaturen und Anlagen eingesetzt werden. Der Verfahrensgang der erfindungsgemäßen Arbeitsweise ist dabei in dem in der Abbildung dargestellten Fließschema in vereinfachter Form wiedergegeben. Das aufzutrennende Einsatzprodukt wird hierbei durch die Leitung 1 im flüssigen Zustand in den mittleren Teil der mit Böden oder sonstigen Einbauten versehenen Extraktivdestillationskolonne 2 eingeleitet. Durch die Leitung 3 wird das Lösungsmittel in die Extraktivdestillationskolonne 2 eingeleitet und fließt über die Einbauten dieser Kolonne herab nach unten, wobei es die Olefine aufnimmt.

Oberhalb der Einmündung der Leitung 3 in die Extraktivdestillationskolonne 2, das heißt oberhalb des Lösungsmittelaufgabebodens, ist der Kolonnenaufsatz 4 angeordnet, der mit der Extraktivdestillationskolonne 2 eine bauliche Einheit bildet, und der ebenfalls mit Böden oder sonstigen Einbauten versehen ist. Die aus der Extraktivdestillationskolonne 2 aufsteigenden Paraffindämpfe gelangen deshalb unmittelbar in den Kolonnenaufsatz 4. Hier werden durch den über die Leitung 16 aufgegebenen Rückfluß die in den Paraffinen noch vorhandenen Lösungsmittelreste ausgewaschen, so daß die Paraffine im reinen Zustand über die Leitung 15 über Kopf abgezogen und ihrer weiteren Verwendung zugeführt werden können. Von der Leitung 15 zweigt dabei die Leitung 16 für den erforderlichen Rückfluß ab. Das flüssige Sumpfprodukt der Extraktivdestillationskolonne 2 besteht aus dem Lösungsmittel und den darin gelösten Olefinen. Es wird durch die Leitung 5 aus der Extraktivdestillationskolonne 2 abgezogen und gelangt in die Abtriebskolonne 6, in der die Olefine destillativ vom Lösungsmittel abgetrennt werden. Das Lösungsmittel wird durch die Leitung 7 aus dem Kolonnensumpf entfernt und gelangt nach entsprechender Abkühlung, die im allgemeinen in einem wirtschaftlichen Wärmeverbund innerhalb der Anlage erreicht wird, über die Leitung 3 wieder in die Extraktivdestillationskolonne 2 zurück, während die Olefindämpfe über Kopf aus der Abtriebskolonne 6 entweichen und durch die Leitung 8 ihrer weiteren Verarbeitung zugeführt werden. Da sich im Laufe der Zeit im Lösungsmittel Verunreinigungen anreichern können, ist im Bereich der Leitung 7 die Abzweigleitung 9 vorgesehen, durch die bei entsprechender Stellung des Ventils 1o eine Teilmenge des Lösungsmittels zur Regeneriereinrichtung 11 gelangen kann. Das regenerierte Lösungsmittel wird durch die Leitung 12 wieder in den Kreislauf (Leitung 7) zurückgeführt, während die ausgeschiedenen Verunreinigungen durch die Leitung 13 aus der Regeneriereinrichtung 11 abgezogen werden. Die Leitung 14 dient schließlich der Zufuhr von frischem Lösungsmittel.

Durch Anwendung des erfindungsgemäßen Verfahrens ist es bei gleich guten Trennergebnissen möglich, eine Einsparung an Betriebs- und Anlagekosten in der Größenordnung von 1o - 15 % gegenüber der bisher bekannten Arbeitsweise zu erzielen, bei der die Raffinatbehandlung in einer separaten Kolonne erfolgt.

**Patentansprüche**

1. Verfahren zur Trennung von paraffinischen und olefinischen $C_4$-Kohlenwasserstoffen durch Extraktivde-

3

stillation unter Verwendung von wasserfreiem Morpholin als selektives Lösungsmittel, wobei die Olefine zusammen mit der Hauptmenge des Lösungsmittels aus dem Sumpf der Extraktivdestillationskolonne (2) abgezogen und in einer nachgeschalteten Abtriebskolonne (6) vom Lösungsmittel abgetrennt werden, während die Paraffine als Kopfprodukt (15) aus der Extraktivdestillationskolonne entfernt werden, dadurch gekennzeichnet, daß die in den Paraffinen enthaltenen Lösungsmittelreste in einem mit der Extraktivdestillationskolonne (2) eine bauliche Einheit bildenden und ebenfalls mit Böden oder sonstigen Einbauten versehenen Kolonnenaufsatz (4) aus den Paraffinen entfernt werden, wobei der Kolonnenaufsatz mit einem Rückflußverhältnis von 1 bis 2 betrieben wird.

## Claims

1. A process for the separation of paraffinic and olefinic $C_4$ hydrocarbons by extractive distillation using anhydrous morpholine as a selective solvent, the olefins being drawn off from the bottom of the extractive distillation column (2), together with the bulk of the solvent, and separated from the solvent in a downstream stripper column (6), while the paraffins are removed from the extractive distillation column as the top product (15), characterised in that the solvent residues contained in the paraffins are removed from the paraffins in a column attachment (4) forming a structural unit with the extractive distillation column (2) and also provided with trays or other built-in parts, the column attachment being operated with a reflux ratio of 1 to 2.

## Revendications

1. Procédé de séparation, par distillation extractive, d'hydrocarbures paraffiniques et oléfiniques en $C_4$, en utilisant de la morpholine anhydre comme solvant sélectif, les oléfines étant extraites du produit de pied de la colonne (2) de distillation extractive avec la majeure partie du solvant et séparées de ce dernier dans une colonne (6) de rectification montée en aval, tandis que les paraffines sont éliminées de la colonne de distillation extractive sous forme de produit de tête (15),
ledit procédé étant caractérisé par le fait que les restes de solvant contenus dans les paraffines sont séparés de celles-ci dans une colonne (4) d'appoint formant une unité constructive avec la colonne (2) de distillation extractive et pourvue également de plateaux ou autres infrastructures, la colonne d'appoint fonctionnant avec un rapport de reflux de 1 à 2.